**Europäisches Patentamt**

**European Patent Office**

**Office européen des brevets**

(19)

(11) Numéro de publication: **0 013 525**
**B1**

(12) **FASCICULE DE BREVET EUROPEEN**

(45) Date de publication du fascicule du brevet: **09.02.83**

(51) Int. Cl.³: **G 01 T 1/164**

(21) Numéro de dépôt: **79401066.0**

(22) Date de dépôt: **21.12.79**

(54) Caméra à scintillation à résolution spatiale améliorée.

(30) Priorité: **09.01.79 FR 7900422**

(43) Date de publication de la demande:
**23.07.80 Bulletin 80/15**

(45) Mention de la délivrance du brevet:
**09.02.83 Bulletin 83/6**

(84) Etats contractants désignés:
**DE GB IT NL**

(56) Documents cités:
**DE - A - 2 653 837**
**DE - B - 1 028 163**
**US - A - 3 708 622**

**MEDICAL RADIOISOTOPE SCINTIGRAPHY, Vol. II, 6—15 août 1968, Salzbourg IAEA Vienne (AT) C. KELLERSHOHN et al.: "Caméra gamma à amplificateur d'image, application en médécine nucléaire" p. 63—74.**

(73) Titulaire: **THOMSON-CSF**
**173, Boulevard Haussmann**
**F-75379 Paris Cedex 08 (FR)**

(72) Inventeur: **Roux, Georges**
**"THOMSON-CSF" - SCPI 173, bld Haussmann**
**F-75360 Paris Cedex 08 (FR)**
Inventeur: **Roussel, Dominique**
**"THOMSON-CSF" - SCPI 173, bld Haussmann**
**F-75360 Paris Cedex 08 (FR)**

(74) Mandataire: **Thrierr, Francoise et al,**
**THOMSON-CSF SCPI 173, Bld Haussmann**
**F-75379 Paris Cedex 08 (FR)**

Courier Press, Leamington Spa, England.

# Caméra à scintillation à résolution spatiale améliorée

La présente invention concerne une amélioration des caméras à scintillation, ou caméras de scintigraphie conformes au préambule de la revendication permettant d'obtenir une amélioration de la résolution spatiale sans, pour autant, détériorer la résolution en énergie de ces caméras.

Les caméras à scintillation sont utilisées pour la détection et la localisation des rayonnements nucléaires, et principalement des rayons gamma, émis par un objet radio-actif qui est généralement un organe humain rendu radioactif par introduction d'un produit lui-même radio-actif. Ces caméras permettent d'obtenir une image de l'organe dans lequel s'est localisé le produit radio-actif et sont particulièrement intéressantes pour les diagnostics.

Il existe actuellement deux principaux types de caméras de scintigraphie. Le premier type, apparu le premier dans l'état de la technique, est la caméra dite de Anger. Le second type de caméras de scintigraphie est la caméra à amplificateur de brillance.

Ces deux types de caméras ont un certain nombre de caractéristiques communes qui font que la présente invention est applicable aussi bien à l'un qu'à l'autre type. Un rapide résumé de ces caractéristiques communes va être fait ici pour permettre de mieux comprendre en quoi consiste l'invention. Une description plus détaillée de ces caméras sera faite à l'occasion de la description donnée plus loin.

Les caractéristiques communes de ces deux types de caméras consistent en la combinaison de trois moyens qui sont les suivants:

Un premier moyen constiue la tête de la caméra détectant les rayons gamma. Cette tête comprend plusieurs éléments: un cristal scintillateur; une fenêtre transparente; une ou plusieurs photo-cathodes.

Le cristal scintillateur, placé dans une enveloppe de protection, reçoit les rayons gamma émis par l'objet radio-actif à observer, et les traduit en scintillations qui consistent en une émission de photons dans toutes les directions à partir du point d'impact du rayon gamma dans le cristal. Les photons ainsi émis par le cristal scintillateur sont transmis par la fenêtre transparente à la (ou aux) photo-cathode qui les dirigera vers des moyens de traitement décrits plus loin.

Il est important de noter que parmi les photons reçus par la photo-cathode certains correspondent à l'information utile; ce sont ceux qui sont engendrés par des rayons gamma directement émis par l'objet radio-actif à examiner. D'autres correspondent à une information parasite; ce sont ceux qui correspondent à des rayons gamma diffusés dans le patient. Ces photons parasites qui sont généralement plus faibles en énergie que ceux correspondant à l'information utile, seront à éliminer. Il est donc important de noter ici qu'il faudra, au niveau du traitement de l'information, pouvoir effectuer une discrimination en énergie des photons. Il est, pour cela, nécessaire que la caméra ait une bonne résolution en énergie.

La fenêtre transparente, à travers laquelle les photons sont transmis vers la (ou les) photocathode, est généralement couplée au cristal par un couplant optique parfaitement transparent qui a pour seule fonction de réaliser une adaptation des indices entre le cristal scintillateur et la fenêtre transparente.

Les photons transmis à travers cette fenêtre transparente sont alors recueillis par une photocathode dans le cas de la caméra à amplificateur de brillance, par plusieurs photocathodes dans le cas de la caméra de Anger. Cette photo-cathode transforme les photons qu'elle reçoit en électrons qui vont être traités pour obtenir l'image souhaitée.

Le deuxième moyen commun aux deux types de caméras consiste en des moyens d'amplification des électrons émis par la (ou les) photocathode. Ces moyens sont, dans le cas de la caméra de Anger, les photo-multiplicateurs correspondant aux photo-cathodes déjà mentionnées. C'est, dans le cas de la caméra à amplificateur de brillance, l'amplificateur lui-même.

Le troisième moyen consiste en un certain nombre de moyens de calcul du centre de gravité de la tache lumineuse correspondant à la scintillation engendrée par le rayon $\gamma$ frappant le cristal. Ces moyens de calcul, connus en eux-mêmes, seront rapidement mentionnés dans la description suivante. Dans les cas de la caméra de Anger ils comportent les photo-multiplicateurs déjà mentionnés dans le deuxième moyen précité; les signaux délivrés par ces photo-multiplicateurs sont ensuite traités dans un dispositif de combinaison électrique de ces signaux, qui permet de calculer le centre de la tache. Dans le cas de la caméra à amplificateur de brillance, ces moyens consistent en un localisateur électronique placé derrière la sortie du tube amplificateur de brillance; ce localisateur délivre des signaux à des circuits de traitement qui permettront d'obtenir l'image souhaitée.

Il est bien connu que les quantités d'information utilisées dans le calcul de la position de la scintillation sont très faibles et soumises aux lois de la statistique. Il s'ensuit que le calcul de la position du centre de gravité d'une tache lumineuse par les moyens de calcul précités, est entaché d'une variance qui dépend d'un certain nombre de paramètres, et qui dépend de façon très importante de la forme et de l'étendue géométrique de la tache lumineuse correspondant à la scintillation. Cette variance se traduit par une certaine imprécision dans la détermination du centre de gravité d'une tache lumineuse. Cette imprécision, qu'il est souhaitable de réduire le plus possible, se mesure généralement par une grandeur appelée résolution spatiale de la caméra. La résolution spatiale peut être définie de la façon suivante. Lorsque les scintillations sont toujours produites au même endroit dans le cristal, la position de chaque scintillation est restituée avec une fluctuation; cette fluctuation est mesurée par la valeur d'une grandeur appelée résolution spatiale, et est généralement exprimée par la largeur à mi-hauteur de la distribution des coordonnées calculées d'un grand nombre de scintillations se produisant toujours au même endroit dans le cristal. Cette distribution est également appelée dans l'état de la technique: fonction de distribution de point, ou fonction de distribution de ligne.

Le but essentiel de la présente invention consiste à améliorer cette résolution spatiale sans pour autant dégrader la résolution en énergie qui, comme déjà expliqué, est un paramètre très important pour obtenir une bonne image visualisée.

Diverses tentatives ont déjà été faites pour tenter d'améliorer cette résolution spatiale. Ces tentatives consistaient par exemple à réaliser certains traitement supplémentaires dans les moyens de calcul. Le problème important qui s'est toujours posé, est celui de réaliser cette amélioration sans qu'elle se traduise par une perte dans la résolution en énergie.

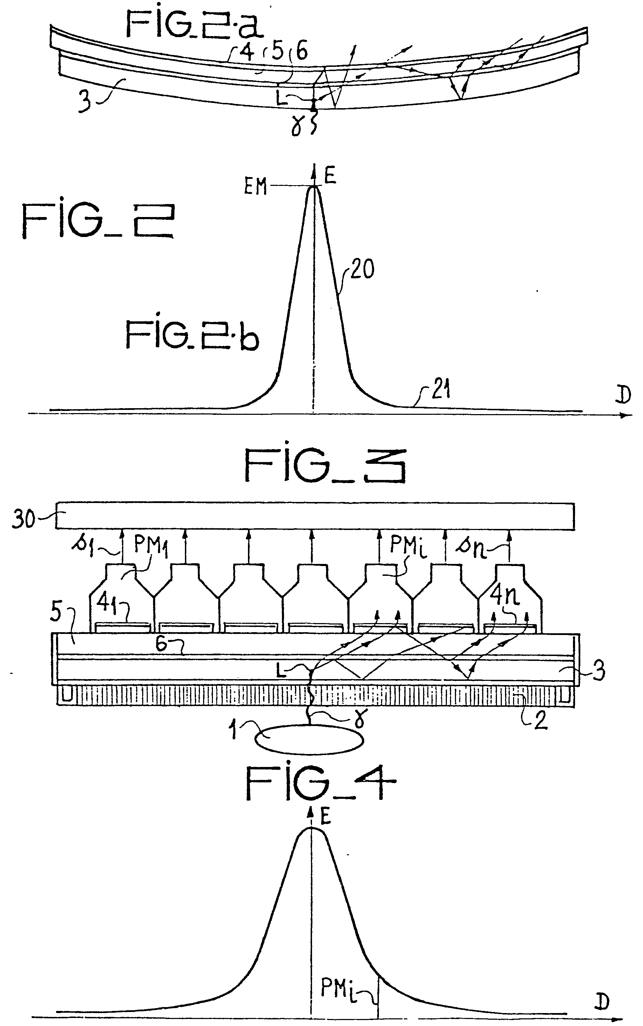

Selon "MEDICAL RADIO ISOTOPE SCINTIGRAPHY", Vol. 11, 6—15 août 1968, Salzbourg IAEA Vienne (AT), dans l'article C. KELLERSOHN et al.: "Caméra gamma à amplificateur d'image, application en médecine nucléaire", figures 3, 4, pages 67, 68, on a aussi résolu le problème d'amélioration de la résolution spatiale en utilisant une matrice de cristaux scintillateurs et en jouant sur un verre interposé entre ceux-ci et la photocathode par suppression du contact optique, ce qui produit une limitation spatiale par effet de réflexion totale, et cela sans perte de lumière et donc de sensibilité dans la résolution énergétique.

Selon DE—A—2 653 837, figures 6, 7, pages 9 à 11, revendication 1, on a aussi tenté d'améliorer la résolution spatiale en choisissant l'épaisseur du cristal en dessous d'une limite de 9 mm tout en gardant un poli grossier de la face antérieure et postérieure du cristal scintillateur.

Selon US—A—3 708 622 et DE—B—1 028 163, l'augmentation du contraste par couche de verre absorbant fortement la lumière, à coefficient d'absorption contrôlé, était en soi connue dans le domaine des tubes cathodiques, et pour des tubes vidicon en particulier. La présente invention vis au perfectionnement susmentionné et résoud le problème technique posé dans une caméra à scintillation selon le préambule de la revendication 1, par les moyens figurant dans la partie caractérisante de la revendication 1, afin de transmettre pratiquement sans absorption des rayons directement issus du cristal et à absorber les rayon diffractes et réfléchis par les différents milieux optiques présents entre le cristal et la photocathode.

Il a été constaté que cela est possible et il sera expliqué plus loin comment la résolution spatiale est ainsi améliorée sans que soit détériorée de façon sensible la résolution en énergie de la caméra.

Des formes d'exécution de l'invention ressortiront de la description suivante, donnée à titre d'exemple non limitatif et illustrée par les figures annexées qui representent:

— la figure 1 une vue schématique d'une caméra à amplificateur de brillance et des moyens de calcul associés, pour l'obtention d'une image représentant un objet radio-actif à examiner, améliorée conformément à l'invention;

— la figure 2 (a) et (b), une vue schématique, en coupe, d'une tête de caméra conforme à la figure 1, où sont schématiquement représentés les trajets optiques d'un certain nombre de rayons lumineux délivrés par le cristal scintillateur (2a), et une courbe donnant l'allure de l'éclairement de la photo-cathode recevant une scintillation en fonction de la distance au centre de la scintillation (2b);

— la figure 3 une vue schématique d'une caméra de scintigraphie du type caméra d'Anger:

— la figure 4 une courbe donnant l'allure de la lumière reçue par les photo-multiplicateurs de la caméra de la figure 3 en fonction de la. distance des photo-multiplicateurs par rapport au centre de la scintillation.

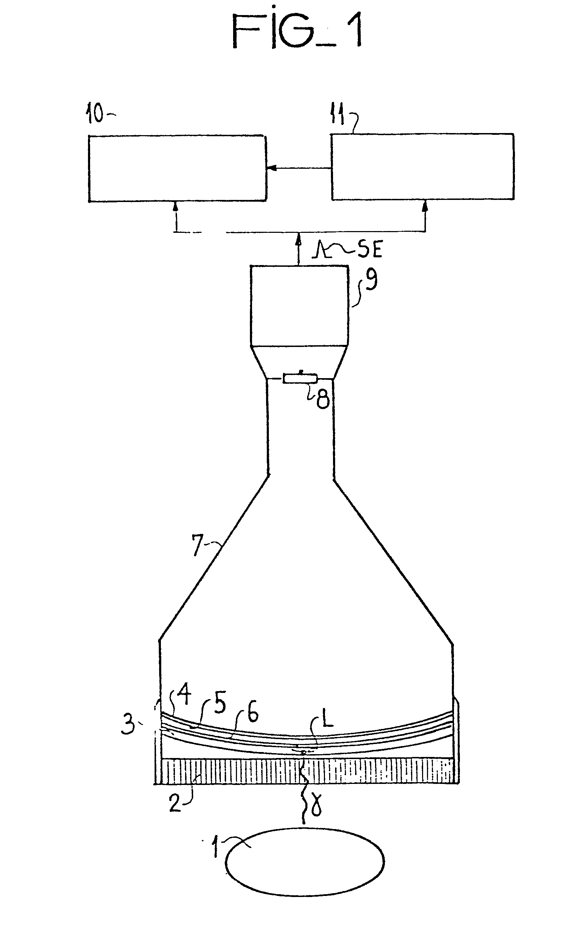

La figure 1 donne le schéma de principe d'une caméra de scintillation à amplificateur de lumière. Les rayons gamma issus de l'objet radioactif 1 sont canalisés par un collimateur 2 généralement en plomb, et atteignent le cristal 3 où ils ont une grande probabilité d'être arrêtés. Le pouvoir d'arrêt du cristal dépend de sa constitution, mais pour le cristal généralement utilisé — iodure de sodium activé au thallium — 90% des rayons gamma de 140 kev sont arrêtés sur 12 mm d'épaisseur. L'effet d'absorption des rayons gamma est essentiellement d'origine photo-électrique, ce qui signifie que toute l'énergie du rayon gamma est transformée en énergie électronique, cette énergie étant elle-même transformée, sous un volume très réduit, inférieur à 100 microns, en une quantité de lumière proportionnelle à l'énergie du rayon gamma incident. La trace du rayon gamma arrêté dans le cristal est donc une scintillation très brève émettant de la lumière dans toutes les directions. Le maximum de cette lumière est capté en couplant le cristal 3 à une photo-cathode 4 généralement déposée sur une fenêtre de verre transparent 5, la liaison optique entre le cristal 3 et la fenêtre de verre 5 se faisant par un couplant optique 6 d'indice relativement proche de celui du verre de la fenêtre d'entrée 5. Les couplants optiques utilisés dans l'art antérieur de la présente invention ont, pour adapter les indices du cristal, généralement de l'ordre de 1, 8, et du verre de l'ordre de 1, 5, un indice compris entre ces deux valeurs. Il est connu que le couplant idéal permettant d'obtenir une bonne adaptation des indices est un couplant dont l'indice est égal à

$$\sqrt{1,5 \times 1,8} = 1,643.$$

Il est important de noter que dans cet état de la technique le couplant, de même que la fenêtre de verre 5, sont choisis pour être le moins absorbant possible et laisser passer la totalité des rayons lumineux, de manière à ne pas altérer l'information utile.

Dans la caméra à scintillation décrite ici, la photo-cathode 4 est celle d'un amplificateur de lumière 7 réducteur d'image avec lequel une lumière de scintillation, amplifiée, est obtenue, à la sortie de l'amplificateur de lumière, sur un écran secondaire 8 couplé à un localisateur électronique 9. L'écran de sortie 8 du tube amplificateur de lumière 7 est couplé au localisateur électronique 9 par tout dispositif du couplage approprié, tel qu'un milieu transparent.

Le localisateur électronique 9 est nécessaire car la lumière de scintillation, à cause de la nécessaire épaisseur du cristal détecteur des rayons gamma, est distribuée sur une tache largement plus grande que les dimensions de la scintillation elle-même. Le localisateur a pour but de restituer le centre de gravité de la tache de lumière sortant du cristal, la position de ce centre de gravité étant homothétique de celle de la position de la scintillation dans le cristal. Nous parlerons, pour la simplification, dans la suite de l'exposé, uniquement de la position de la tache lumineuse de scintillation, sachant qu'en fait il s'agit de la position du centre de gravité calculée par le localisateur électronique. Différents types de localisateurs électroniques sont connus de l'homme de l'art; ils ne seront pas décrits ici. Ils peuvent être constitués par exemple par quatre photo-multiplicateurs convertissant la lumière qu'ils reçoivent de l'écran secondaire 8 du tube amplificateur de lumière 7, en signaux électroniques appliqués aux moyens de calcul déjà cités. Ils peuvent être constitués par des dispositifs à semiconducteur comme décrit par exemple dans la demande de brevet français FR—A—2 402 880 du 9 septembre 1977 déposée par la Compagnie THOMSON-CSF. Les signaux électriques SE délivrés par ce localisateur électronique 9 vont permettrent d'une part de calculer comme déjà dit la position du centre de gravité de la tache lumineuse grâce à des moyens de calcul 10 classiques en eux-mêmes et comportant à leur sortie un dispositif de visualisation. Ils vont être utilisés d'autre part dans un dispositif de spectrométrie permettant de réaliser une sélection en énergie des rayons gamma pour permettre comme expliqué précédemment d'éliminer les rayons gamma diffusés par le corps du sujet examiné. Comme l'énergie lumineuse fournie par le cristal de la tête de caméra est proportionnelle à l'énergie du rayon gamma incident et comme les amplificateurs de lumière et le localisateur sont des dispositifs linéaires, les signaux électriques fournis par le localisateur sont proportionnels aux énergies gamma. Une combinaison particulière, et classique en elle-même, de ces signaux, effectuée dans un dispositif de sélection d'énergie 11 permet de commander la visualisation des scintillations uniquement si l'énergie gamma mesurée est dans une bande d'énergie choisie. Plus le pic photo-électrique des énergies gamma primaire est étroit, meilleure est l'élimination du rayonnement diffusé. C'est pour cette raison que, comme il à déjà été expliqué, il est indispensable de ne pas détériorer la résolution en énergie de la caméra, sous prétexte de vouloir améliorer sa résolution spatiale. Il peut être noté que la résolution en énergie, exprimée comme la largeur à mi-hauteur du pic photo-électrique du spectre des énergies gamma est souvent donnée en pourcentage de l'abscisse du pic photo-électrique.

Les figures 2a et 2b permettent de bien comprendre le perfectionnement de la présente invention. La figure 2a représente de façon plus détaillée la tête de caméra de scintigraphie. Elle donne le détail des différents milieux optiques rencontrés par la lumière dûe à une scintillation L créée dans le cristal 3 par un rayon gamma incident. Le chemin optique de certains rayons lumineux issus de la scintillation a été dessiné pour montrer comment la tache lumineuse atteigant la photo-cathode peut avoir une distribution d'éclairement très étendue. L'étendue de cette tache apparaît clairement sur la figure 2b qui représente l'éclairement E de la photo-cathode 4 en fonction de la distance D au centre de la scintillation supposée se produire ici au centre de la tête de caméra. Il apparaît clairement sur cette figure 2b que la tache lumineuse comporte d'une part une partie centrale 20 très lumineuse, ayant son maximum $E_M$ situé au centre de la scintillation, et une partie latérale — ou queue — 21 de très faible intensité et s'étalant tout autour de la partie centrale 20. L'examen des chemins lumineux de la figure 2a montre que cet étalement très important de la tache lumineuse est dû aux effets de dioptres multiples séparant les milieux d'indices de réfraction différents comme ceux du cristal et de la fenêtre d'entrée, associés à une photo-cathode qui est toujours plus où moins réfléchissante. Cet effet de dioptres et de réflexions multiples conduit à une diffusion de la lumière sur des régions très éloignées de la scintillation, cette diffusion s'effectuant principalement par des rayons ayant une grande obliquité.

Des expériences faites par la demanderesse prouvent que la résolution spatiale est considérablement améliorée si la partie latérale 21 de la tache lumineuse peut être éliminée. La présente invention consiste en un moyen permettant d'éliminer cette partie latérale — ou queue 21, ou tout au moins de la réduire de façon très importante.

Le moyen permettant de pratiquement éliminer cette queue de la tache 21 consiste à disposer, sur le trajet des rayons lumineux issus de la scintillation 1, un absorbant lumineux à absorption contrôlée.

Dans un exemple de réalisation, cet absorbant consiste en l'utilisation d'un couplant optique 6 qui n'est plus, comme c'était le cas dans l'art antérieur, complètement transparent, mais qui présente un coefficient d'absorption de la lumière non négligeable.

Si e cm est l'épaisseur du couplant et que son coefficient d'absorption de la lumière est $\mu$ cm$^{-1}$, on peut démontrer que la transmission d'un flux lumineux émis sous un angle d'incidence $\theta$ est:

$$T(\theta) = \exp - \frac{\mu e}{\cos \theta}$$

Cette formule montre que la transmission des rayons lumineux, pour un couplant d'épaisseur e et de coefficient d'absorption $\mu$, n'est fonction que de l'angle des rayons lumineux. En particulier quand l'angle $\theta$ est très important, c'est-à-dire pour des rayons obliques créateurs de la queue de la tache lumineuse, la transmission s'annule car $\cos \theta$ devient très petit et

$$\frac{\mu e}{\cos \theta}$$

très grand.

Ainsi en utilisant un couplant optique 6 qui n'est plus complètement transparent comme 1' étaient ceux de l'art antérieur, mais qui présente un coefficient d'absorption lumineux $\mu$ non négligeable, l'énergie lumineuse utile, c'est-à-dire celle de la partie centrale 20 de la tache lumineuse est très peu modifiée, et celle de la queue 21 est très diminuée par l'effet d'absorption pour les rayons obliques ayant un angle $\theta$ relativement important. On a ainsi réalisé une absorption lumineuse contrôlée. La partie utile de la tache correspondant à des rayons lumineux peu inclinés est pratiquement conservée intacte, tandis que la partie latérale 21 correspondant à des rayons lumineux inclinés ou très inclinés, est considérablement réduite. Il en résulte comme précédemment indiqué une amélioration importante de la résolution spatiale. La résolution en énergie, quant à elle, n'est pas dégradée puisque la partie centrale de la tâche est conservée pratiquement intacte.

Il est également possible de réaliser cette absorption contrôlée non plus en utilisant un couplant optique à absorption contrôlée, mais en utilisant une fenêtre d'entrée 5 présentant ces propriétés.

Diverses solutions peuvent être utilisées pour réaliser ce couplant optique, ou cette fenêtre, à absorption de lumière contrôlée.

Un mode de réalisation particulièrement intéressant de l'invention consiste à utiliser un couplant optique connu en lui-même, qui est par exemple une résine transparente souple, dans laquelle se trouve noyée une poudre d'aluminium de granulo-métrie prédéterminée, la quantité de poudre noyée dans le couplant étant elle-même prédéterminée de manière à obtenir le coefficient d'absorption approprié. Les grains d'aluminium agissent comme des obstacles sur le trajet des rayons lumineux et l'absorption de la lumière s'effectue selon le processus connu en lui-même de "section efficace d'absorption", cette section efficace d'absorption étant fonction de la dimension des grains d'aluminium.

L'utilisation d'une poudre d'aluminium présente de plus l'avantage que l'aluminium réfléchit la lumière vers sa direction d'origine, produisant ainsi un effet catodioptrique qui est bénéfique.

L'absorption contrôlée conforme à l'invention est réalisée de la même manière avec toute autre poudre absorbante de granulométrie prédéterminée. Elle peut également être réalisée avec un absorbant uniformément réparti dans la masse du couplant, comme un pigment coloré. Le couplant optique peut être autre chose qu'une résine souple, à partir du moment où l'uniformité et la stabilité du pouvoir absorbant en fonction de l'angle $\theta$ sont conservées.

Comme il a déjà été dit l'absorption contrôlée peut être réalisée non plus au niveau du couplant optique, mais de la fenêtre d'entrée 5 qui est par exemple réalisée, pour cela, en un verre teinté.

La demanderesse a réalisé une caméra à scintillation à amplificateur de lumière conforme à la présente invention. L'amélioration de résolution spatiale obtenue a été vérifiée et a permis de construire une caméra à grand champ d'entrée sans perte de résolution spatiale. Ainsi, dans un premier essai, une résolution spatiale de 4,2 millimètres a été obtenue, pour des rayons gamma de 140 kev du Technétium avec une résolution en énergie de 10% sur un champ utile de 32 cm. Ces valeurs ne sont données qu'à titre d'exemple et ne sont pas l'expression des limites qui pourraient être atteintes, mais elles sont à comparer avec les résultats déjà publiés et montrent les améliorations dûes à la présente invention.

La figure 3 représente très schématiquement une caméra de Anger. La tête de caméra comporte les éléments déjà décrits à propos de la caméra à amplificateur de brillance. Il s'agit d'un collimateur 2 à travers lequel passent les rayons gamma émis par l'objet radio-actif 1; d'un cristal scintillateur épais 3 protégé par une enveloppe dont la partie arrière constitue la fenêtre transparente 5; la liaison entre le cristal 3 et la fenêtre étant faite par un couplant optique 6.

La photo-cathode 4 du tube amplificateur de lumière 7 est ici remplacée par les photo-cathodes $4_1$, $4_2$ ... $4i$ ... $4n$ des n photomultiplicateurs $PM_1$, $PM_2$ ... $PM_n$ de la caméra de Anger.

La différence essentielle entre la caméra à amplificateur de brillance décrite précédemment et la caméra de Anger est que les photomultiplicateurs $PM_1$ ... $PM_n$ assurent à la fois la fonction d'amplificateur qui était réalisée par le tube 7, et la fonction de localisateur qui était réalisée par le localisateur électronique 9. Les n photomultiplicateurs délivrent n signaux électriques $s_1$ ... $s_i$ ... $s_n$ qui sont appliqués et combinés dans un dispositif 30 de combinaison électrique. Ce dispositif permet la restitution de la position de la scintillation. Rien dans ce dispositif n'étant modifié par la présente invention, il n'est pas besoin de le décrire ici.

La courbe de la figure 4 indique, comme le faisait celle de la figure 2b, la quantité de lumière E reçu par les divers photomultiplicateurs en fonction de leur distance par rapport au centre de la scintillation L créée dans le cristal 3 par le rayon gamma incident. Le même raisonnement que celui qui a été fait à propos de la caméra à amplificateur de lumière peut être fait ici. Les parties latérales de la courbe de distribution de la lumière reçue par les divers photo-multiplicateurs conduisent à une grande variance de la détermination des coordonées de la scintillation. L'utilisation de la présente invention, c'est-à-dire la disposition sur le trajet des rayons lumineux délivrés par le cristal scintillateur 3, d'un absorbant à absorption contrôlée comme précédemment décrit, conduit à une amélioration de la résolution spatiale sans dégrader la résolution en énergie. En effet, les rayons diffractés et réfléchis étant absorbés comme il a été précédemment expliqué, les photo-multiplicateurs éloignés de la scintillation n'amènent plus leur contribution néfaste dans le calcul de la position de la scintillation.

## Revendications

1. Caméra à scintillation pour la détection et la localisation de rayonnements nucléaires, et notamment de rayons $\gamma$ émis par un objet (1) à examiner, comportant une tête de caméra où un cristal scintillateur (3), recevant les rayons $\gamma$, les transforme en scintillations engendrant des rayons lumineux transmis, à travers une fenêtre transparente (5) à une ou plusieurs photocathodes ($4$; $4_1$ ... $4_n$), et comportant des moyens d'amplification et de traitement des informations émises par la ou les photocathodes de manière à calculer le centre de gravité de la tache lumineuse de chaque scintillation, caractérisée en ce que la tête de caméra comporte, entre la sortie du cristal scintillateur (3) et l'entrée de la ou des photocathodes ($4$; $4_1$ ... $4_n$) des moyens à absorption lumineuse contrôlée traitant les rayons lumineux à leur sortie du cristal (3) de manière à les absorber d'autant plus qu'ils sont plus inclinés par rapport à la normale à la photocathode respective, c'est-à-dire à transmettre pratiquement sans absorption les rayons directement issus du cristal (3) et à absorber les rayons diffractés et réfléchis par les différents milieux optiques présents entre cristal (3) et la ou les photo-cathodes ($4$; $4_1$ ... $4_n$).

2. Caméra à scintillation selon la revendication 1, caractérisée en ce que les moyens à absorption contrôlée sont obtenus en répartissant de manière uniforme, dans au moins un des milieux optiques séparant le cristal scintillateur (3) de la (ou des) photo-cathodes ($4$; $4_1$ ... $4_n$), un matériau à coefficient d'absorption lumineuse prédéterminé.

3. Caméra à scintillation selon la revendication 2, caractérisée en ce que, le cristal scintillateur (3) étant couplé à la fenêtre transparente (5) de la tête de caméra par un couplant optique (6) adaptant les indices de réfraction du cristal et de la fenêtre, le matériau à coefficient d'absorption prédéterminé est réparti dans la masse du couplant optique (6).

4. Caméra à scintillation selon la revendication 3, caractérisée en ce que le couplant optique (6) étant constitué par une résine transparente, le matériau à coefficient d'absorption prédéterminé consiste en une poudre absorbant la lumière, de granulomètrie prédéterminée, noyée dans le couplant optique (6).

5. Caméra à scintillation selon la revendication 4, caractérisée ne ce que la poudre est une poudre d'aluminium.

6. Caméra à scintillation selon la revendication 2, caractérisée en ce que le matériau à coefficient d'absorption prédéterminé est réparti dans la masse de la fenêtre transparente (5).

7. Caméra à scintillation selon la revendication 2, caractérisée en ce que le matériau à coefficient d'absorption prédéterminé est constitué par un pigment coloré.

## Patentansprüche

1. Scintillationskamera zur Detektion und Lokalisierung von Kernstrahlung, insbesondere von $\gamma$-Strahlung, die von einem zu untersuchenden Objekt (1) emittiert wird, mit einer Aufnahmekamera, in der ein die $\gamma$-Strahlen empfangender Scintillationskristall (3) diese in Scintillationen umsetzt, welche Lichtstrahlen erzeugen, die durch ein lichtdurchlässiges Fenster (5) zu einer (oder mehreren) Photokatoden ($4$; $4_1$ ... $4_n$) übertragen werden, und mit Einrichtungen zur Verstärkung und derartigen Verarbeitung der von den Photokatoden ausgesandten Informationen, daß der Schwerpunkt des Lichtfleckes jeder Scintillation berechnet wird, dadurch gekennzeichnet, daß die Aufnahmekamera zwischen dem Ausgang des Scintillatorkristalls (3) und dem Eingang der Photokatoden (4; $4_1 \ldots 4_n$) Lichtabsorptionsmittel mit gesteuerter Absorption umfaßt, welche die Lichtstrahlen bei ihrem Austreten aus dem Kristall (3) derart behandeln, daß sie umso stärker absorbiert werden, je größer ihre Neigung in Bezug auf die Senkrechte zu der Photokatode ist, daß sie also praktisch ohne Absorption die von dem Kristall (3) direkt ausgehenden Strahlen durchlassen und die von den verschiedenen optischen Medien zwischen dem Kristall (3) und den Photokatoden (4; $4_1 \ldots 4_n$) gebrochenen und reflektierten Strahlen absorbieren.

2. Scintillationskamera nach Anspruch 1, dadurch gekennzeichnet, daß die Absorptionsmittel mit gesteuerter Absorption erhalten werden, indem in wenigstens einem der optischen Medien, die den Scintillatorkristall (3) von der (oder den) Photokatode (n) (4; $4_1 \ldots 4_n$) trennen, ein Material gleichmäßig verteilt wird, welches einen vorbestimmten Lichtabsorptionskoeffizienten aufweist.

3. Scintillationskamera nach Anspruch 2, dadurch gekennzeichnet, daß, wenn der Scintillationskristall (3) an das lichtdurchlässige Fenster (5) der Aufnahmekamera über ein optisches Kopplungsmittel (6) angekoppelt ist, welches die Brechungsindices des Kristalles und des Fensters aneinander anpaßt, das Material mit vorbestimmten Absorptionskoeffizienten in der Masse des optischen Kopplungsmittels (6) verteilt ist.

4. Scintillationskamera nach Anspruch 3, dadurch gekennzeichnet, daß, wenn das optische Kopplungsmittel (6) aus einem lichtdurchlässigen Harz gebildet ist, das Material mit vorbestimmten Absorptionskoeffizienten aus einem lichtabsorbierenden Pulver vorbestimmter Korngröße besteht, das in das optische Kopplungsmittel (6) eingebettet ist.

5. Scintillationskamera nach Anspruch 4, dadurch gekennzeichnet, daß das Pulver ein Aluminiumpulver ist.

6. Scintillationskamera nach Anspruch 2, dadurch gekennzeichnet, daß das Material mit vorbestimmten Absorptionskoeffizienten in der Masse des lichtdurchlässigen Fensters (5) verteilt ist.

7. Scintillationskamera nach Anspruch 2, dadurch gekennzeichnet, daß das Material mit vorbestimmtem Absorptionskoeffizienten durch ein Farbpigment gebildet ist.

**Claims**

1. Scintillation camera for the detection and localization of nuclear radiation, particularly $\gamma$ rays emitted by an object (1) to be examined, comprising a camera head wherein a scintillation crystal (3) receiving the $\gamma$ rays transforms these into scintillations generating light rays transmitted through a transparent window (5) to one (or a plurality of) photo-cathodes (4; $4_1 \ldots 4_n$), and comprising amplification and processing means for processing the information issued by the photo-cathodes in a manner to calculate the center of gravity of the light spot of each scintillation, characterized in that, between the output of the scintillation crystal (3) and the input of the photo-cathodes (4; $4_1 \ldots 4_n$), the camera head comprises light absorption means having controlled absorption and treating the light rays exiting from the crystal (3) in a manner to absorb them so much the more the more they are inclined with respect to the normal on the photo-cathode, i.e. to transmit the rays directly emerging from the crystal (3) practically without absorption and to absorb the rays diffracted and reflected by the different optic media between the crystal (3) and the photo-cathodes (4; $4_1 \ldots 4_n$).

2. Scintillation camera in accordance with claim 1, characterized in that the controlled absorption means are obtained by uniform distribution of a material having a predetermined light absorption coefficient in at least one of the optic media separating the scintillation crystal (3) from the photo-cathode(s) (4; $4_1 \ldots 4_n$).

3. Scintillation camera in accordance with claim 2, characterized in that, the scintillation crystal (3) being coupled to the transparent window (5) of the camera head by an optic coupling medium (6) adapting the refraction indices of the crystal and the window, the material having a predetermined absorption coefficient is distributed within the mass of the optic coupling medium (6).

4. Scintillation camera in accordance with claim 3, characterized in that the optic coupling medium (6) being formed by a transparent resin, the material having a predetermined absorption coefficient consists of a light absorbing powder of predetermined grain size, embedded into the optic coupling medium (6).

5. Scintillation camera in accordance with claim 4, characterized in that the powder is an aluminum powder.

6. Scintillation camera in accordance with claim 2, characterized in that the material having a predetermined absorption coefficient is distributed within the mass of the transparent window (5).

7. Scintillation camera in accordance with claim 2, characterized in that the material having a predetermined absorption coefficient is formed by a color pigment.

0 013 525

# FIG_1

SE

1

0 013 525

FIG.2·a

FIG_2

FIG.2·b

FIG_3

FIG_4